(19) Europäisches Patentamt
European Patent Office
Office européen des brevets

(11) Numéro de publication: **0 076 755**
**B1**

(12) # FASCICULE DE BREVET EUROPÉEN

(45) Date de publication du fascicule du brevet: **13.01.88**

(21) Numéro de dépôt: **82401793.3**

(22) Date de dépôt: **01.10.82**

(51) Int. Cl.⁴: **C 07 D 471/18**, A 61 K 31/435 // (C07D471/18, 221:00, 221:00, 221:00)

(54) **Acylamino-4 aza-1 adamantanes, procédé pour leur préparation, et application en thérapeutique.**

(30) Priorité: **05.10.81 FR 8118682**

(43) Date de publication de la demande: **13.04.83 Bulletin 83/15**

(45) Mention de la délivrance du brevet: **13.01.88 Bulletin 88/2**

(84) Etats contractants désignés: **AT BE CH DE FR GB IT LI LU NL SE**

(56) Documents cité: **FR-A-2 358 404**

(73) Titulaire: **ETABLISSEMENTS NATIVELLE S.A., 27, rue de la Procession, F-75015 Paris (FR)**

(72) Inventeur: **Jarreau, François- Xavier, 5, rue Louis Hervé, F-78000 Versailles (FR)**
Inventeur: **Koenig, Jean- Jacques, 31 rue du Panorama, F-77670 Vernou La Celle s/Seine (FR)**

(74) Mandataire: **L'Helgoualch, Jean, OFFICE PICARD 134 Boulevard de Clichy, F-75018 Paris (FR)**

EP 0 076 755 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention, réalisée dans les laboratoires du CERES-Centre Européen de Recherche Scientifique - concerne de nouveaux dérivés de l'adamantane, et plus particulièrement des dérivés de la série acylamino-4 aza-1 adamantane, leur application en thérapeutique, ainsi qu'un procédé pour leur préparation.

On connait divers composés comprenant dans leur molécule un noyau de type adamantyle, dont la structure à quatre cycles hexagonaux condensés peut entrainer diverses proprietés physiques et chimiques particulières en raison de sa rigidité stérique. Les dérivés de type aza-adamantane, ou aza-tricyclo (3,3,1) décane, présentant un groupe adamantyle où un atome d'azote est substitué à un atome de carbone en tête de pont, à la jonction de trois des cycles condensés du noyau adamantyle tétracyclique, ont été très peu étudiés. Un exemple de méthode de préparation de tels dérivés du type aza-adamantane notamment l'amino-4 aza-1 triméthyl-4,8,8 adamantane et son dérivé acétylé, est décrit dans le brevet français 2 358 404.

L'invention a pour objet de nouveaux acylamino-4 triméthyl-4,8,8 aza-1 adamantanes susceptibles d'être utilisés en thérapeutique pour le traitement des maladies cardiovasculaires.

L'invention a également pour objet un procédé de préparation de nouveaux dérivés du type acylamino-4 triméthyl-4,8,8 aza-1 adamantane.

L'objet de la présente invention s'étend aussi aux nouveaux médicaments constitués par des dérivés du type acylamino-4 triméthyl-4,8,8 aza-1 adamantane, ainsi qu'aux compositions pharmaceutiques les contenant, pour le traitement des maladies cardiovasculaires.

Les nouveaux acylamino-4 aza-1 adamantanes conformes à la présente invention peuvent être représentés par la formule générale (I) ci-dessous:

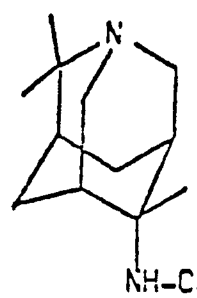

(I)

dans laquelle R représente un groupe phényle, un groupe naphtyle, un groupe tolyle, un groupe phénéthyle, un groupe benzyle, un groupe phénylpropyle, un groupe diphényl-2,2 éthyle, ou un groupe diphényl-3,3 propyle, ces groupes pouvant porter un ou plusieurs substituants choisis parmi un atome d'halogène ou un groupe alkyle de 1 à 4 atomes de carbone, hydroxy, méthylènedioxy, méthoxy, éthoxy, isopropoxy, amino, isopropylamino, diméthylamino, diéthylamino, nitro cyano, acétylamino, formyle, acétyle, trifluorométhyle et trichlorométhyle, ainsi que leur sels d'acides.

Le groupe aryle représenté par R peut porter un ou plusieurs substituants choisis parmi un atome d'halogène ou un groupe alkyle (par exemple méthyle, éthyle, propyle, butyle), hydroxy, méthylènedioxy, alcoxy (par exemple méthoxy, éthoxy, isopropoxy, etc), amino, alkylamino (par exemple isopropylamino), dialkylamino (par exemple diméthylamino, diéthylamino), nitro, cyano, acylamino (par exemple acétylamino), formyle, acétyle, halogénométhyle (par exemple trifluorométhyle, trichlorométhyle), pour former par exemple un groupe p-nitrophényle, p-aminophényle, p-acétylaminophényle, p-méthoxyphényle, p-méthoxyphénéthyle, diméthoxy-3,4 phényle, diméthoxy-3,4 phénéthyle, diméthoxy-3,4 benzyle, dihydroxy-3,4 phényle, p-chlorophényle, dichloro-3,4 benzyle, méthylènedioxy-3,4 phényle, méthylènedioxy-3,4 benzyle, p-trichlorométhylphényle, p-trifluorométhylbenzyle, p-cyanophényle, p-cyanophénéthyle, cyano-2 diphényl-2,2 éthyle, cyano-3 diphényl-3,3 propyle.

L'invention concerne de préférence les composés de formule générale (I) dans laquelle R représente un groupe aryle tel qu'un groupe phényle, benzyle, phénéthyle, phénylpropyle, diphényl-2,2 éthyle et diphényl-3,3 propyle, ou un groupe aryle substitud tel qu'un groupe p-nitrophényle, p-aminophényle, p-acétylaminophényle, p-méthoxyphényle, diméthoxy-3,4 phényle, diméthoxy-3,4 benzyle, p-méthoxybenzyle, p-méthoxyphénéthyle et méthylènedioxy-3,4 phényle.

L'invention concerne également les sels des dérivés du type acylamino-4 aza-1 adamantane, représentés par la formule générale (I) ci-dessus, et en particulier les sels pharmaceutiquement acceptables, obtenus en faisant réagir un acide minéral ou organique sur le dérivé de formule (I) comme base. Cette réaction de salification peut s'effectuer suivant les méthodes usuelles de la technique, en faisant réagir l'acide et le dérivé du type acylamino-4 aza-1 adamantane de formule (I) en proportions sensiblement stoechiométriques, dans un solvant approprié tel que le méthanol, l'éthanol, l'isopropanol, le tétrahydrofuranne, le dioxanne, le chlorure de méthylène, l'éther éthylique, l'acétate d'éthyle, etc. L'acide peut être par exemple l'acide chlorhydrique, l'acide lactique, l'acide tartrique, l'acide phosphorique, l'acide oxalique, l'acide formique, l'acide sulfurique, l'acide maléique, l'acide bromhydrique, l'acide iodhydrique, etc.

Les nouveaux acylamino-4 aza-1 triméthyl-4,8,8 adamantanes suivant l'invention peuvent être obtenus à partir da l'amino-4 aza-1 triméthyl-4,8,8 adamantane de formule générale (II) ci-dessous:

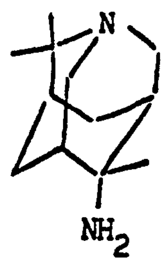

par action d'un agent d'acylation, dans un solvant approprié.

La réaction d'acylation peut être effectuée suivant les techniques usuelles, par exemple au moyen d'un acide, un chlorure d'acide, un ester ou un anhydride. On peut utiliser notamment un agent d'acylation choisi parmi l'anhydride acétique, l'anhydride propionique, l'acide N-diéthylamino acétique (ou diéthylglycine), l'acide N-diméthylamino acétique, le chlorure de benzoyle, le chlorure d'acide p-nitrobenzoique, le chlorure d'acide p-méthoxybenzoique, l'acide phénylacétique, l'acide phényl-3 propionique, l'acide diphényl-3,3 propionique, l'acide phénylbutyrique, etc. L'agent d'acylation est de préference utilisé en léger excès.

Tous les solvants utilisés couramment dans les réactions d'acylation peuvent convenir pour la préparation des dérivés conformes à l'invention, notamment un éther tel que l'éther diéthylique, la tétrahydrofuranne, le dioxanne, un solvant chloré tel que le tétrachlorure de carbone, le chloroforme, le chlorure de méthylène, ou un ester tel que l'acétate d'éthyle. Suivant l'invention on utilise de préference le chlorure de méthylène et le chloroforme.

La réaction d'acylation de l'amino-4 aza-1 triméthyl-4,8,8 adamantane de formule (II) s'effectue à froid, et il peut être avantageux de dissoudre les produits de départ dans un solvant refroidi sur bain de glace ou sur bain d'eau froide, et de laisser la température s'élever lentement au cours de la réaction.

Afin de faciliter la réaction d'acylation, notamment lorsque l'agent d'acylation est un acide tel que l'acide phénylacétique, l'acide phénylbutyrique, etc., il est avantageux d'ajouter au milieu réactionnel du N-hydroxysuccinimide et du dicyclohexylcarbodiimide. Les quantités utilisées peuvent être par exemple de l'ordre de 1 à 2 moles de N-hydroxysuccinimide et de 1 à 3 moles de dicyclohexylcarbodiimide pour 1 à 2 moles d'acide et 1 mole d'amino-4 aza-1 triméthyl-4,8,8 adamantane de formule (II).

Le cas échéant, la base obtenue peut être salifiée, comme indiqué ci-dessus, ou transformée par modification d'un substituant. Par exemple le dérivé de formule (I) où R est un groupe p-nitrobenzoyle, obtenu par action du chlorure d'acide p-nitrobenzoique sur le dérivé de formule (II), peut être réduit par hydrogénation sur catalyseur pour le transformer en dérivé correspondant où R est un groupe p-aminobenzoyle qui peut lui-même être acétylé en groupe p-acétylaminobenzoyle par les techniques usuelles, par exemple par action du chlorure d'acétyle dans le tétrahydrofuranne.

L'amino-4 aza-1 triméthyl-4,8,8 adamantane de formule générale (II) est un produit connu, décrit dans le brevet français 2 358 404, que l'on peut préparer à partir d'un pinène traité par un sel mercurique et un nitrile en milieu anhydre pour obtenir une imine bicyclique qui est réduite en amine, puis cyclisée par action d'un aldéhyde pour procurer l'azaadamantane de formule (II).

Les exemples donnés ci-après illustrent l'invention plus en détail sans en limiter la portée.

**Exemple 1**

N-(p-aminobenzoyl) amino-4 triméthyl-4,8,8 aza-1 adamantane.

On dissout 6,8 g d'amino-4 triméthyl-4,8,8 aza-1 adamantane dans 100 ml de chloroforme, en refroidissant la solution sur bain de glace, et on ajoute goutte à goutte 7,3 g de chlorure d'acide p-nitrobenzoïque dissous dans 70 ml de chloroforme.

Après réaction, on recueille par filtration le précipité formé, que l'on dissout dans l'ammoniaque. On extrait le phase aqueuse par le chlorure de méthylène pour procurer 5,59 de N-(p-nitrobenzoyl) amino-4 triméthyl-4,8,8 aza-1 adamantane, sous forme de cristaux blancs dont le point de fusion est: F = 206°C

Spectre I.R. (Nujol) ν = 3420, 3200, 1650, 1600, 1570, 1520 $cm^{-1}$

CCM ($CH_2Cl_2$, MeOH, $NH_4OH$, 80-20-1) Rf = 0,60

Par réduction du produit ci-dessus par l'hydrogène en présence de platine dans une solution à 10 % de méthanol, puis filtration et recristallisation dans l'isopropanol, on obtient le N-(p-aminobenzoyl) amino-4 triméthyl-4,8,8 aza-1 adamantane sous forme de cristaux blancs.

Point de fusion: F = 218°C

Spectre I.R. (Nujol) ν = 3440, 3320, 1640, 1610 $cm^{-1}$

CCM ($CH_2Cl_2$-MeOH-$NH_4OH$, 80-20-1) Rf = 0,40

Le ces échéant, on peut acétyler le produit ci-dessus en le dissolvant dans du tétrahydrofuranne, et en ajoutant goutte à goutte une solution de chlorure d'acétyle dans le tétrahydrofuranne; après recristallisation dans l'éthanol, on obtient le N-(p-acétylaminobenzoyl) amino-4 triméthyl-4,8,8 aza-1 adamantane sous forme

de chlorhydrate.

| | |
|---|---|
| Chlorhydrate: | F = 230°C (éthanol) |
| Spectre I.R. (Nujol) | ν = 3650 à 2000, 1670, 1630, 1610, 1600, 1530, 1510cm$^{-1}$ |
| Base: | |
| Spectre IR (Nujol) | ν = 3600 à 2000, 1670, 1640, 1600, 1535, 1505 cm$^{-1}$ |
| C.C.M. (AcoOEt + 20 % $HNEt_2$) | Rf=0,20 |

**Exemple 2**

N-(phényl-3 propionyl) amino-4 triméthyl-4,8,8 aza-1 adamantane

On fait réagir 15,6 g d'acide phényl-3 propionique sur 12,5 g d'amino-4 triméthyl-4,8,8 aza-1 adamantane en présence de 6,2 g de N-hydroxysuccinimide et 24,0 g de dicyclohexylcarbodiimide dans 130 ml de chlorure de méthylène pendant 72 h.

Après filtration, traitement et séparation de la dicyclohexylurée formée, purification par les techniques usuelles et cristallisation dans l'acétate d'étnyle, on obtient 13,7 g de N-(phényl-3 propionyl) amino-4 triméthyl-4,8,8 aza-1 adamantane (rendement 65 %).

| | |
|---|---|
| Point de fusion: | F = 134 - 136°C (Acétate d'éthyle) |
| Spectre IR (Nujol): | ν = 2800 à 3500 (3230 et 3050), 1645, 1600, 1565, 1490, 755 et 700 cm$^{-1}$ |
| Spectre de RMN ($CDCl_3$) | δ = 1,25 (6H, s), 1,49 (3H, s), 1,0 à 2,3 (7H), 2,5 (2H, m) 2,9 (2H, m), 3,0 (2H, d, J=15), 3,45 (2H, d, J=15), 5,35 (1H mobile), 7,35 (5H) ppm. |
| CCM ($CH_2Cl_2/MeOH/NH_4OH$ 84/16/3) | Rf= 0,55 |

On dissout 4,5 g de la base ci-dessus dans 30 ml de tétrahydrofuranne, on décante le précipité gluant, on le dissout dans 15 ml d'éthanol absolu et on coule la solution dans 100 ml d'éther isopropylique sous agitation au bain de glace. Après filtration, 5,9 g de tartrate de N-azaadamantyl phényl propionamide sont obtenus (rendement 89 %)

Point de fusion: F = 95 - 110° % (fusion pâteuse) (Ethanol/éther isopropylique)

**Exemple 3**

N-(p-méthoxyphényl-3 pyropionyl) amino-4 triméthyl-4,8,8 aza-1 adamantane.

On fait réagir 8,4 g d'acide p-méthoxyphényl-3 propionique sur 6,0 g d'amino-4 triméthyl-4,8,8 aza-1 adamantane, en présence de 4,3 g de N-hydroxy succinimide et de 13,6 g de dicyclohexylcarbodiimide, dans 80 ml de chlorure de méthylène pendant 40 h.

On extrait la fraction basique par les techniques usuelles et on cristallise le résidu dans de l'acétate d'éthyle. On obtient ainsi 5,6 g de N-(p-méthoxyphényl-3 propionyl) amino-4 triméthyl-4,8,8 aza-1 adamantane avec un rendement de 51 %.

| | |
|---|---|
| Point de fusion: | F = 149 - 151°C (acétate d'éthyle) |
| CCM ($CH_2Cl_2/MeOH/NH_4OH$ 85/15/2) | Rf= 0,55 |
| Spectre IR (Nujol) | ν = 3000 à 3600 (maximum vers 3290), 1635, 1610, 1550, 1510 cm$^{-1}$. |
| Spectre de RMN ($CDCl_1$) | δ = 1,23 (6H, s), 1,48 (3H, s), 1,0 à 2,3 (7H), 2,50 (2H, m), 2,90 (2H, m), 2,98 (2H, d, J=15), 3,45 (2H, d, J=15), 3,75 (3H, s) 5,60 (1H mobile), 6,77 (2H, d, J=9), 7,13 (2H, d, J=9) ppm. |

On transforme la base ci-dessus en chlorhydrate correspondant par les techniques usuelles, par action de l'acide chlorhydrique concentré dans l'éthanol.

| | |
|---|---|
| Point de fusion: | F = > 260°C (éthanol) |

**Exemple 4**

N-(diphényl-3,3 propionyl) amino-4 triméthyl-4,8,8 aza-1 adamantane.

On fait réagir 8,4 g d'acide diphényl-3,3 propionique en deux fractions de 6,4 g puis de 2 g, sur 4,5 g d'amino-4 triméthyl-4,8,8 aza-1 adamantane en présence de 3,5 g de N-hydroxy succinimide et de 9,5 g de dicyclohexylcarbodiimide dans 75 ml de chlorure de méthylène pendant 48 h.

Après traitement et élimination de la dicyclohexylurée formée, par les techniques usuelles, on obtient 6,4 g de résidu cristallin que l'on purifie par cristallisation dans un mélange d'acétate d'éthyle et d'éthanol.

| | |
|---|---|
| Point de fusion: | F = 222 - 224°C (éthanol/acétate d'éthyle) |
| Spectre IR (Nujol) | ν = 3285, 1660, 1635, 1595, 1550, 1540, 700 et 690 cm$^{-1}$ |

Spectre de RMN ($CDCl_3$) δ = 1,23 (6H, s), 1,38 (3H, s), 0,9 à 2,3 (7H), 2,90 (2H, d, J=8), 2,93 (2H, d, J=15), 3,35 (2H, d, J=15), 4,50 (1H, t, J=8), 5,35 (1H mobile), 7,20 (10H, s) ppm.

CCM ($CH_2Cl_2/MeOH/NH_4OH$ 85/15/2) Rf = 0,4

On dissout 6,4 g de base brute obtenue comme indiqué ci-dessus dans 100 ml de tétrahydrofuranne bouillant. On ajoute 2,5 g d'acide L(+)tartrique dissous dans 25 ml de tétrahydrofuranne chaud. On laisse refroidir, on filtre et on recristallise le précipité obtenu (8,4 g) dans l'éthanol absolu pour procurer 7,0 g de tartrate de N-(diphényl-3,3 propionyl) amino-4 triméthyl-4,8,8 aza-1 adamantane (rendement 80 %),

Point de fusion: F = 224 - 228° C (éthanol)

**Exemples 5 à 9**

On procède comme indiqué dans l'exemple 1, en remplaçant le chlorure d'acide p-nitrobenzoïque par le chlorure de benzoyle, ou par le chlorure d'acide p-méthoxybenzoïque, et on obtient respectivement le N-benzoylamino-4 triméthyl-4,8,8 aza-1 adamantane (exemple 5), ou le N-(p-méthoxybenzoyl) amino-4 triméthyl-4,8,8 aza-1 adamantane (exemple 6).

De même, en procédant comme dans l'exemple, mais en remplaçant l'acide phényl-3 propionique par l'acide phényl-2 acétique, ou par l'acide diméthoxy-3,4 phénylacétique, ou par l'acide phényl-4 butyrique, on obtient respectivement le N-phénylacétyl amino-4 triméthyl-4,8,8 aza-1 adamantane (exemple 7) ou le N-(diméthoxy-3',4' phénylacétyl) amino-4 triméthyl-4,8,8 aza-1 adamantane (exemple 8), ou le N-(phényl-4 butyryl) amino-4 triméthyl-4,8,8 aza-1 adamantane (exemple 9), dont les caractéristiques sont indiquées au tableau ci-après.

| Exemple n° | F (°C) (solvant) | Spectre IR ($cm^{-1}$) (Nujol) | Sel | F (°C) |
|---|---|---|---|---|
| 5 | 130-132 ($H_2O$) | - | chlorhyd. | > 260 |
| 6 | 110-120 (AcOEt) | 2500-3500,1630,1600 1555,1500,1245 | chlorhyd. | > 260 |
| 7 | 169-171 (AcOEt) | 3300,1655,1635,1545 725 | tartrate | 100-110 |
| 8 | 169-171 (AcOEt) | 3290,1640,1610,1595 1545,1515,1265,1235 1160,1030,790 | chlorhyd. | > 260 |
| 9 | 62-64 (AcOEt + $(i\text{-}C_3H_7)_2O$) | 3000-3500,1640,1565 1490,740,695 | chlorhyd. | > 260 |

Les expérimentations effectuées sur les acylamino-4 aza-1 adamantanes conformes à la présente invention ont fait apparaître d'intéressantes propriétés toxicologiques et pharmacologiques, qui démontrent leur application possible en thérapeutique humaine et vétérinaire.

**Etude toxicologique**

La toxicité aigüe des dérivés de l'invention a été étudiée par administration intrapéritonéale (I.P.) à la souris (10 animaux, 5 mâles et 5-femelles par dose) et calcul de la dose léthale 50 (DL 50) selon la méthode de Litchfield et Wilcoxon (J. Pharmacol. 1949, 96 99-113). Le tableau 1 présente les valeurs de DL 50 des dérivés dont la préparation est décrite dans les exemples 1 à 9.

Dans certains cas (dérivés des exemples 2, 4, 7), la DL 50 a été également calculée après administration orale (P.O.) des dérivés.

**Tableau 1**

DL 50 par voieintrapéritonéale et par voie orale

| Exemple n° | DL 50 I.P. (mg/kg) | DL 50 P.O. (mg/kg) |
|---|---|---|
| 1 | 95 | |
| 2 | 375 | 2400 |
| 3 | 250 | |
| 4 | 51 | 380 |
| 5 | 230 | |
| 6 | 250 | |
| 7 | 430 | 2400 |

**Propriétés pharmacologiques**

A. Tolérance hémodynamique

La tolérance hémodynamique des dérivés de l'invention a été étudiée chez le chien anesthésié au pentobarbital sodique. Les pressions endocavitaires sont meaurées à l'aide de cathéters reliés à des capteurs Statham tandis que l'enregistrement de l'électrocardiogramme (E.C.G.) externe permet la mesure de la fréquence cardiaque. Le débit cardiaque est mesuré à l'aide d'un capteur électromagnétique placé sur l'aorte à son origine. Les résistances périphériques totales R sont calculées à partir de la valeur de la pression aortique moyenne ($\bar{P}$) et du débit cardiaque (Q) selon la relation $R = \bar{P}/Q$.

Après mesure des paramètres en période contrôle, les dérivés sont injectés par voie intraveineuse en doses cumulatives (30 minutes d'intervalle entre les doses). Les variations des paramètres par rapport à la période contrôle sont mesurées entre 20 et 30 minutes après chaque injection et exprimées en pourcentage de variation par rapport au contrôle.

Le tableau 2 résume les variations des paramètres hémodynamiques observées entre la première et la dernière injection.

**Tableau 2**

Tolérance Cardiovasculaire chez le Chien Anesthésié

(pourcentages de variation par rapport à période contrôle)

| Exemple n° | Doses cumulatives mg/kg | Pression artérielle systolique | Fréquence cardiaque | Débit cardiaque | dp/dt/p ventriculaire gauche | Résistances périphériques totales |
|---|---|---|---|---|---|---|
| 1 | 0,1 à 3% | - 3 à -33% | 0 à -14% | +7 à -32% | +14 à -50% | -10 à + 7% |
| 2 | 0,3 à 10 | - 7 à -28% | - 7% | -7 à -23% | 0 à -40% | 0 à -13% |
| 3 | 1 à 10 | 0 à + 3% | 0 | +4 à - 8% | + 2 à -15% | - 6 à +11% |
| 4 | 0,1 à 1 | - 8 à -16% | -3 à +10% | -3 à -18% | - 6 à -13% | 0 |
| 6 | 0,3 à 10 | - 6 à -21% | -7 à -15% | -7 à -22% | 0 à -28% | 0 |
| 7 | 1 à 10 | - 5 à -28% | 0 à -12% | 0 à -30% | 0 à -33% | - 5 à +15% |

Cette étude de tolérance hémodynamique montre que:

- La pression artérielle systolique diminue de 16 à 35 % avec tous les dérivés excepté le dérivé de l'exemple 3 qui ne produit pas de modification significative de ce paramètre (0 à +3 %)
- La fréquence cardiaque diminue modérément (maximum -15 % avec le dérivé de l'exemple 6) avec la majorité des dérivés de la série excepté le dérivé de l'exemple 3 où la fréquence ne change pas et celui de l'exemple 4 où elle augmente légèrement (+ 10 %).
- Le débit cardiaque est diminué constamment mais ces variations restent limitées de -8 à -32 %.
- Le rapport de la dérivée première de la pression ventriculaire gauche à la pression ventriculaire gauche instantanée diminue aux fortes doses de -13 à -50 %.
- Les résistances périphériques totales varient peu.

En conclusion, la tolérance cardiovasculaire chez le chien anesthésié est satisfaisante puisque les effets sont limités à une chute modérée du débit cardiaque, de l'indice de contractilité et de la pression artérielle systolique tandis que la fréquence cardiaque et les résistances périphériques totales varient diversement.

B. Propriétés antiarythmiques expérimentales

a) Etudes electrophysiologiques chez le chien anesthésié

L'étude est réalisée chez le chien anesthésié au pentobarbital, à thorax fermé, à l'aide de cathéters-électrodes bipolaires introduits dans les cavités cardiaques par voies veineuse et artérielle transcutanées. L'électrocardiogramme de surface (dérivation standard $D_2$) est enregistré en permanence.

A l'aide d'un stimulateur programmable JANSEN (R) on peut mesurer les paramètres suivants:

- fréquence cardiaque spontanée (FC);
- temps de récupération du sinus (SRT) après stimulation auriculaire imposé à 160 b/mn pendant 1 mn;
- les temps ce conduction intra-cardiaque (auriculohisien à fréquence constante, His-Purkinje, intraventriculaire);
- les périodes réfractaires effectives et fonctionnelles mesurées à fréquence constante imposée, avec la méthode de l'extrastimulus.

Lors de l'étude électrophysiologique, les dérivés sont injectés par voie intraveineuse en 2 minutes pour chaque dose, et à 30 minutes d'intervalle entre chaque dose. Les doses sont exprimées en valeur cumulative et en terme de base.

La mesure des différents paramètres est effectuée avant l'injection de la première dose (période controle) et de 10 à 28 minutes après l'injection de chaque dose de la substance. Les résultats sont exprimés en pourcentages de variation par rapport à la période contrôle.

Les effets électrophysiologiques de quatre exemples de dérivés de l'invention sont présentés dans le tableau 3.

Les résultats obtenus montrent que les dérivés de l'invention ont des effets modérés (ou inconstants) sur l'automaticité sinusale alors qu'ils produisent un allongement constant des temps de conduction intracardiaque à tous les niveaux, ainsi qu'un allongement des périodes réfractaires. De par ces caractères, les dérivés de l'invention produisent chez le chien anesthésié des effets typiques du groupe I de la classification de Vaughan-Williams (groupe de la quinidine et dérivés).

b) Tests Antiarythmiques

L'activité antiarythmique a été observée chez la souris au moyen du test de Lawson suivant la méthode décrite par J.W. Lawson, J. Pharmacol. Exp. Ther. 1968, 160, 22-31, et Cr. Narcisse et al., Ann. Pharma. Fr. 1979, 37, 325-330, chez le rat par le test de l'intoxication à l'aconitine selon S. Witchitz et al., Coeur Méd. Int. 1971, X(2), 281-286 et sur le chien par le test de Harris décrit dans Circulation, 1950, 1, 1318, et par le test à l'adrénaline après infarctus expérimental selon I. J. Steffe et al., J. Pharmacol. Exp. Ther. 1980, 214, 50-57.

Intoxication à l'aconitine

Le rat anesthésié est intoxiqué par une perfusion intraveineuse d'aconitine tandis que son électrocardiogramme (ECG) est enregistré en permanence. Durant la perfusion à vitesse constante, on mesure le temps nécessaire à l'apparition d'arythmies ventriculaires, successivement extra-systoles (ESV), puis tachycardie ventriculaire (TV) stable et le temps au bout duquel survient la mort de l'animal.

**Tableau 3**

Effets Electrophysiologiques

| Dérivés n° | Nombre Expér. | Doses (mg/kg) | Variation (%) | | | | | |
|---|---|---|---|---|---|---|---|---|
| | | | F.C. | St-H | HV | QRS | PREA | PREV |
| 1 | 3 | 0,5 à 5 | 0 à - 24 | +2 à +76 | +13 à +98 | +8 à +39 | + 2 à +50 | 0 à +20 |
| 2 | 2 | 0,3 à 14,3 | 0 | +7 à +10 | 0 à + 80 | 0 à +15 | +10 à +35 | 0 à +10 |
| 4 | 1 | 0,à 1,4 | 0 à +35 | 0 à + 25 | 0 à + 92 | 0 à +50 | +18 à +50 | +10 à +20 |
| 7 | 2 | 1 à 19 | 0 | 0 à +30 | +18 à +100 | 0 à +23 | + 6 à +45 | + 2 à +15 |

F.C.: Fréquence cardiaque
St-H: Temps de conduction auriculo-hisien
HV: Temps de conduction His-Purkinje
QRS: Temps de conduction intraventriculaire
PREA: Période réfractaire effective auriculaire
PREV: Période réfractaire effective ventriculaire

Les animaux sont répartis en lot témoin (non traité) et lots traités (différentes doses). Les résultats sont exprimés en pourcentage d'allongement du délai d'apparition des arythmies et de la mort dans les lots traités

par rapport au lot témoin.

Les résultats obtenus sont regroupés au tableau 4 ci-après, et expriment le pourcentage d'allongement du délai d'apparition des arythmies ventriculaires (extrasystoles ventriculaires et tachycardies ventriculaires), et de la mort, induites par l'aconitine après une injection intraveineuse (I.V.) de plusieurs dérivés conformes à l'invention, par rapport à un groupe d'animaux témoins non traités.

**Tableau 4**

Arythmies ventriculaires

| Dérivé n° | Dose injectée mg/kg | ESV (%) | TV (%) | Mort (%) |
|---|---|---|---|---|
| 6 | 5 | + 45 | + 43 | + 46 |
| | 10 | + 71 | + 84 | +215 |
| | 20 | + 98 | +107 | +112 |
| 4 | 1 | + 52 | + 93 | + 69 |
| | 2 | + 67 | +139 | + 97 |
| 7 | 10 | + 31 | + 79 | + 44 |
| | 20 | +100 | + 79 | + 84 |
| | 40 | +171 | +147 | + 88 |
| 2 | 5 | + 37 | + 50 | + 25 |
| | 10 | + 60 | + 86 | + 39 |
| | 20 | +103 | + 89 | + 65 |
| 3 | 5 | + 41 | + 20 | + 21 |
| | 10 | + 51 | + 48 | + 36 |
| | 20 | + 65 | + 54 | + 50 |
| 5 | 5 | + 59 | + 53 | + 29 |
| | 10 | + 35 | + 35 | + 45 |
| | 20 | + 111 | +105 | + 89 |

Les résultats indiqués au tableau 4 montrent que les dérivés suivant l'invention exercent un effet protecteur contre les arythmies puisqu'ils allongent nettement le délai d'apparition des arythmies ventriculaires et de la mort.

Test de Lawson

Le test de Lawson est un test d'étude du pouvoir antifibrillatoire cardiaque des dérivés. Les souris (20 par dose) reçoivent une injection intrapéritonéale du dérivé 10 minutes avant d'être placées en atmosphère saturée en chloroforme. Dès l'arrêt respiratoire obtenu, le thorax est ouvert (5 à 10 secondes) et on vérifie si le coeur est ou non en fibrillation ventriculaire. La dose efficace 50 (DE 50) du dérivé étudié est la dose qui protège la moitié des souris contre la fibrillation ventriculaire anoxique.

Le tableau 5 résume les résultats du test de Lawson obtenus avec certains dérivés conformes à la présente invention.

**Tableau 5**

| Dérivé n° | DE 50 (mg/kg) | Dose maxima tolérée (mg/kg) |
|---|---|---|
| 1 | 37 | 50 |
| 2 | 72 | 200 |
| 3 | 61 | 200 |
| 5 | 76 | 150 |
| 6 | 49 | 100 |
| 7 | 90 | 200 |

Ces résultats montrent que les dérivés suivant l'invention possèdent une activité antifibrillatoire satisfaisante, comparable à celle d'un composé connu tel que la quinidine.

Test de Harris

La ligature de l'artère interventriculaire antérieure chez le chien anesthésié entraine l'apparition d'un infarctus du myocarde expérimental suivi d'importantes arythmies ventriculaires.

Les chiens sont étudiés 24 à 48h après l'intervention. L'électrocardiogramme (ECG) est alors enregistré en

permanence, le chien conscient étant au repos dans un laboratoire isolé. Après une période de 3 h d'enregistrement de l'ECG permettant de mesurer la fréquence cardiaque du chien et la fréquence des extrasystoles ventriculaires (ESV) - période contrôle pré-traitement - le dérivé à étudier est injecté par voie intraveineuse en 1 minute. L'enregiatrement continu de l'ECG permet, dans les heures suivant l'injection de mesurer la fréquence des ESV/mn sur des périodes successives de 30 à 60 mn. Le nombre d'ESV par minute mesuré sur 3 h de période de contrôle varie de 66 à 172/mn avec une moyenne à 111/mn. On constate que les dérivés suivant l'invention entraînent une diminution importante du nombre d'ESV par minute, de l'ordre de 35 % à 95 % selon les dérivés et la dose administrée (1 à 14 mg/kg). La durée de cet effet antiarythmique observé varie selon les dérivés de 1 h à plus de 5 h. Par exemple, le pourcentage de diminution des ESV/mn dans le cas du dérivé de l'exemple 9 (dose cumulative 14 mg/kg) est de 96 % (0 à 1/2 h), 50 % (1/2 à 1 h) et 68 % (1 à 2 h). Les valeurs correspondantes pour le dérivé de l'exemple 4, 2 (3 mg/kg) sont respectivement 44 %, 55 % et 45 %.

Arythmies ventriculaires à l'adrénaline après infarctus expérimental

2 à 5 jours après un infarctus expérimental, les effets d'injections discontinues (en bolus intraveineux de 4 g/kg d'adrénaline base) d'adrénaline sont observés sur l'électrocardiogramme du chien conscient.

Au cours d'une première phase (contrôle) de l'expérience, trois injections successives d'adrénaline sont effectuées et on mesure, dans les 2 minutes suivant chaque injection, la fréquence des extrasystoles ventriculaires. On détermine ainsi, en augmentant au besoin la dose d'adrénaline, la dose qui provoque l'apparition d'extrasystoles ventriculaires en fréquence supérieure ou égale à la moitié du nombre total de systoles ventriculaires.

Après injection du dérivé à tester la même dose d'adrénaline est réinjectée à 5 minutes, puis 30 minutes, 60 minutes, 90 minutes, etc., et on compare ses effets à ceux observés en période contrôle.

Le tableau 6 résume les résultats obtenus avec le test à l'adrénaline. Les résultats y sont exprimés en pourcentace d'extrasystoles (sur le nombre total de systoles) mesuré pendant les 2 minutes qui suivent l'injection d'adrénaline avant et après l'administration du dérivé à tester. Ce tableau montre que les effets antiarythmiques peuvent se maintenir plus d'une heure et peuvent aller jusqu'à disparition presque complète des arythmies déclenchées par l'adrénaline.

**Tableau 6**

Test à l'adrénaline après infarctus expérimental

| Exemple n° | Période contrôle (% d'ESV) | Dose injectée (mg/kg) | Pourcentage d'ESV à 5 mn | 30 mn | 60 mn |
|---|---|---|---|---|---|
| 1 | 94 % | 4,3 | 63 % | 77 % | 90 % |
| 2 | 74 % | 14,3 | 36 % | 59 % | 60 % |
| 3 | 40 % | 10 | 9 % | 14 % | 16 % |
| 4 | 85 % | 1,3 | 29 % | 17 % | 17 % |
| 6 | 71 % | 4 | 35 % | 36 % | 53 % |
| 7 | 48 % | 1 | 11 % | 27 % | 45 % |

On constate donc que les dérivés du type acylamino-4 aza-1 adamantane faisant l'objet de la présente invention présentent des propriétés antiarythmiques intéressantes sur différents modèles expérimentaux, qu'il s'agisse d'intoxication à l'aconitine, ou de l'ischémie myocardique aigüe. Leur tolérance cardiovasculaire est bonne car les effets hémodynamiques néfastes restent limités. L'étude de leurs propriétés électrophysiologiques cardiaques montre que ces dérivés possèdent des caractéristiques "quinidine-like" du groupe I des antiarythmiques, et qu'ils agissent aussi bien au niveau supraventriculaire que ventriculaire lui-même, ce qui permet d'envisager des potentialités antiarythmiques étendues.

Ces propriétés montrent que les dérivés suivant l'invention sont utilisables en thérapeutique humaine et vétérinaire, notamment pour le traitement de maladies cardiovasculsires, et plus particulièrement pour le traitement de diverses formes d'arythmies cardiaques, supraventriculaires et ventriculaires.

Les dérivés du type acylamino-4 aza-1 adamantane suivant l'invention et leurs sels pharmeceutiquement acceptables peuvent être administrés sous les formes usuelles, le principe actif étant dilué dans un support pharmaceutiquement acceptable convenablement choisi, par exemple sous forme de comprimé, gélule, dragée, suppositoire, soluté injectable ou sirop.

A titre d'exemple, les comprimés peuvent être préparés en mélangeant le dérivé de formule générale (I) ou un de ses sels, avec un ou plusieurs diluants solides tels que le lactose, le mannitol, l'amidon, la polyvinylpyrrolidone, le stéarate de magnésium, le talc, etc. Le cas échéant, les comprimés peuvent comporter plusieurs couches superposées autour d'un noyau, suivant les techniques usuelles, pour assurer une libération progressive ou un effet retardé du principe actif. L'enrobage peut par exemple être constitué d'une ou plusieurs couches d'acétate de polyvinyle, de carboxyméthylcellulose ou d'acétophtalate de cellulose.

On peut également administrer le dérivé suivant l'invention sous forme d'un sirop ou d'un soluté buvable obtenu en dissolvant le dérivé de formule (I) ou un de ses sels pharmaceutiquement acceptables, dans de l'eau ou du glycérol, par exemple, et en ajoutant le cas échéant un additif usuel tel qu'un édulcorant et un antioxydant.

Des solutions injectables peuvent être préparées suivant les techniques bien connues et sont constituées par exemple par un soluté contenant un dérivé de formule (I) ou un de ses sels pharmaceutiquement acceptables, dissous dans de l'eau bidiatillée, une solution hydroalcoolique, du propylèneglycol, etc., ou un mélange de ces solvants. Le cas échéant, un additif approprié tel qu'un conservateur peut être ajouté.

La posologie peut varier suivant la nature de l'affection traitée, et le sujet concerné. Les doses administrées journellement sont généralement comparables à celles des traitements quinidiniques, mais peuvent être ajustées par le praticien en fonction des circonstances.

**Revendications** pour les Etats contractants: BE, CH, DE, FR, GB, IT, LI, LU, NL, SE.

1. Acylamino-4 aza-1 adamantanes caractérisés en ce qu'ils sont représentés par la formule générale (I):

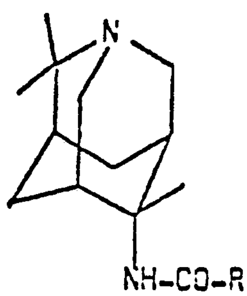

(I)

dans laquelle R représente un groupe phényle, un groupe naphtyle, un groupe tolyle, un groupe phénéthyle, un groupe benzyle, un groupe phénylpropyle, un groupe diphényl-2,2 éthyle, ou un groupe diphényl-3,3 propyle, ces groupes pouvant porter un ou plusieurs substituants choisis parmi un atome d'halogène ou un groupe alkyle de 1 à 4 atomes de carbone, hydroxy, méthylènedioxy, méthoxy, éthoxy, isopropoxy, amino, isopropylamino, diméthylamino, diéthylamino, nitro, cyano, acétylamino, formyle, acétyle, trifluorométhyle et trichlorométhyle, ainsi que leurs sels d'acides.

2. Acylamino-4 aza-1 adamantanes selon la revendication 1, caractérisés en ce que R représente un groupe phényle, benzyle, phénéthyle, phénylpropyle, diphényl-2,2 éthyle, diphényl-3,3 propyle, p-nitrophényle, p-aminophényle, p-acétylaminophényle, p-méthoxyphényle, diméthoxy-3,4 phényle, diméthoxy-3,4 benzyle, p-méthoxybenzyle, p-méthoxyphénéthyle ou méthylène-dioxy-3,4 phényle.

3. Acylamino-4 aza-1 adamantanes selon l'une quelconque des revendications précédentes, caractérisés en ce qu'ils sont sous forme de sels d'acides.

4. Procédé de préparation d'acylamino-4 aza-1 adamantanes selon la revendication 1, caractérisé en ce que l'on traite l'amino-4 aza-1 triméthyl-4,8,8 adamantane de formule générale (II):

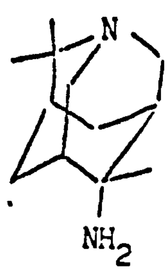

(II)

par action d'un agent d'acylation, dans un solvant approprié.

5. Procédé selon la revendication 4 caractérisé en ce que la réaction d'acylation est effectuée su moyen d'un acide, un chlorure d'acide, un ester ou un anhydride.

6. Procédé selon la revendication 4, caractérisé en ce que la réaction d'acylation s'effectue au moyen d'un acide en présence de N-hydroxysuccinimide et de dicyclohexylcarbodiimide.

7. Procédé selon l'une quelconque des revendications 4 à 6, caractérisé en ce que le solvant est un éther, le tétrahydrofuranne, le dioxanne, le tétrachlorure de carbone, le chloroforme, le chlorure de méthylène, ou un ester.

8. Compositions pharmaceutiques caractérisées en ce qu'elles contiennent un acylamino-4 aza-1 adamantane selon l'une quelconque des revendications 1 à 4, ou un de ses sels pharmaceutiquement acceptables, en tant que principe actif, associé le cas échéant à un support approprié.

**Revendications** pour l'Etat contractant: AT.

1. Procédé de préparation d'acylamino-4 aza-1 adamantanes représentés par la formule générale (I):

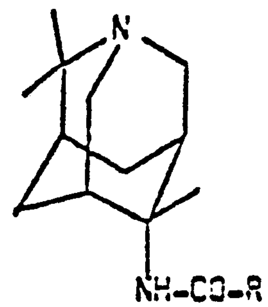

(I)

dans laquelle R représente un groupe phényle, un groupe naphtyle, un groupe tolyle, un groupe phénéthyle, un groupe benzyle, un groupe phénylpropyle, un groupe diphényl-2,2 éthyle, ou un groupe diphényl-3,3 propyle, ces groupes pouvant porter un ou plusieurs substituants choisis parmi un atome d'halogène ou un groupe alkyle de 1 à 4 atomes de carbone, hydroxy, méthylènedioxy, méthoxy, éthoxy, isopropoxy, amino, isopropylamino, diméthylamino, diéthylamino, nitro, cyano, acétylamino, formyle, acétyle, trifluorométhyle et trichlorométhyle, caractérisé en ce que l'on traite l'amino-4 aza-1 triméthyl-4,8,8 adamantane de formule générale (II):

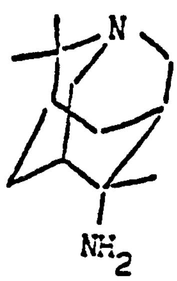

(II)

par action d'un agent d'acylation, dans un solvant approprié.

2. Procédé selon la revendication 1, caractérisé en ce que la réaction d'acylation est effectuée au moyen d'un acide, un chlorure d'acide, un ester ou un anhydride.

3. Procédé selon la revendication 1, caractérisé en ce que la réaction d'acylation s'effectue au moyen d'un acide en présence de N-hydroxysuccinimide et de dicyclohexylcarbodiimide.

4. Procédé selon l'une quelconque des revendications 1 à 3, caractérisé en ce que le solvant est un éther, le tétrahydrofuranne, le dioxanne, le tétrachlorure de carbone, le chloroforme, le chlorure de méthylène, ou un ester.

**Patentansprüche** für die Vertragsstaaten BE, CH, DE, FR, GB, IT, LI, LU, NL, SE.

1. 4-Acylamino-1-aza-adamantane, dadurch gekennzeichnet, das sie der allgemeinen Formel (I) entsprechen:

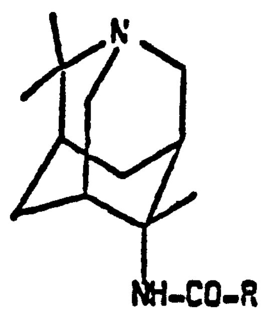

(I)

worin R eine Phenylgruppe, Naphthylgruppe, Tolylgruppe, Phenethylgruppe, Benzylgruppe, Phenylpropylgruppe, 2,2-Diphenylethylgruppe oder eine 3,3-Diphenylpropylgruppe bedeutet, wobei diese Gruppen einen oder mehrere Substituenten tragen können, gewählt aus einem Halogenatom oder einer Alkylgruppe mit 1 bis 4 Kohlenstoffatomen, Hydroxy, Methylendioxy, Methoxy, Ethoxy, Isopropoxy, Amino, Isopropylamino, Dimethylamino, Diethylamino, Nitro, Cyano, Acetylamino, Formyl, Acetyl, Trifluormethyl und

Trichlormethyl sowie deren Säuresalze.

2. 4-Acylamino-1-aza-adamantane nach Anspruch 1, dadurch gekennzeichnet, daß R eine Phenyl-, Benzyl-, Phenethyl-, Phenylpropyl-, 2,2-Diphenylethyl-, 3,3-Diphenylpropyl-, p-Nitrophenyl-, p-Aminophenyl-, p-Acetylaminophenyl-, p-Methoxyphenyl-, 3,4-Dimethoxyphenyl-, 3,4-Dimethoxybenzyl-, p-Methoxybenzyl, p-Methoxyphenethyl- oder 3,4-Methylendioxyphenylgruppe bedeutet.

3. 4-Acylamino-1-aza-adamantane nach einem der vorangehenden Ansprüche, dadurch gekennzeichnet, daß sie als Säuresalze vorliegen.

4. Verfahren zur Herstellung der 4-Acylamino-1-aza-adamantane nach Anspruch 1, dadurch gekennzeichnet, daß man das 4-Amino-1-aza-4,8,8-trimethyl-adamantan der allgemeinen Formel (II)

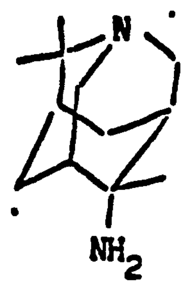

.(II)

in einem geeigneten Lösungsmittel mit einem Acylierungsmittel behandelt.

5. Verfahren nach Anspruch 4, dadurch gekennzeichnet, daß die Acylierungsreaktion mittels einer Säure, einem Säurechlorid, einem Ester oder einem Anhydrid durchgeführt wird.

6. Verfahren nach Anspruch 4, dadurch gekennzeichnet, daß die Asylierungsreaktion mittels einer Säure in Gegenwart von N-Hydroxysuccinimid und Dicyclohexylcarbodiimid durchgeführt wird.

7. Verfahren nach einem der Ansprüche 4 bis 6, dadurch gekennzeichnet, daß das Lösungsmittel ein Ether, Tetrahydrofuran, Dioxan, Kohlenstofftetrachlorid, Chloroform, Methylenchlorid oder ein Ester ist.

8. Arzneimittel, dadurch gekennzeichnet, daß es ein 4-Acylamino-1-aza-adamantan nach einem der Ansprüche 1 bis 3 oder ein pharmazeutisch annehmbares Salz hiervon als Wirkstoff enthält, gegebenenfalls in Verbindung mit einem geeigneten Träger.

**Patentansprüche** für den Vertragsstaat AT.

1. Verfahren zur Herstellung von 4-Acylamino-1-aza-adamantanen der allgemeinen Formel (I)

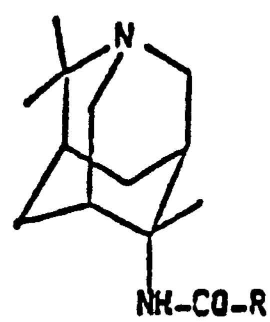

(I)

worin R eine Phenylgruppe, Naphthylgruppe, Tolylgruppe, Phenethylgruppe, Benzylgruppe, Phenylpropylgruppe, 2,2-Diphenylethylgruppe oder eine 3,3-Diphenylpropylgruppe bedeutet, wobei diese Gruppen einen oder mehrere Substituenten tragen können, gewählt aus einem Halogenatom oder einer Alkylgruppe mit 1 bis 4 Kohlenstoffatomen, Hydroxy, Methylendioxy, Methoxy, Ethoxy, Isopropoxy, Amino, Isopropylamino, Dimethylamino, Diethylamino, Nitro, Cyano, Acetylamino, Formyl, Acetyl, Trifluormethyl und Trichlormethyl,

dadurch gekennzeichnet, daß man das 4-Amino-1-aza-4,8,8-trimethyl-adamantan der Formel (II)

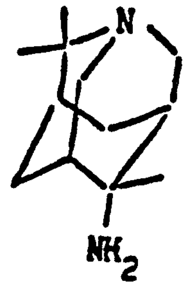

(II)

in einem geeigneten Lösungsmittel mit einem Acylierungsmittel behandelt.

2. Verfahren nach Anspruch 1, dadurch gekennzeichnet, daß die Acylierungsreaktion mittels einer Säure, einem Säurechlorid, einem Ester oder einem Anhydrid durchgeführt wird.

3. Verfahren nach Anspruch 1, dadurch gekennzeichnet, daß die Acylierungsreaktion mittels einer Säure in Gegenwart von N-Hydroxysuccinimid und Dicyclohexylcarbodiimid durchgeführt wird.

4. Verfahren nach einem der Ansprüche 1 bis 3, dadurch gekennzeichnet, daß das Lösungsmittel ein Ether, Tetrahydrofuran, Dioxan, Kohlenstofftetrachlorid, Chloroform, Methylenchlorid oder ein Ester ist.

**Claims** for the Contracting States BE, CH, DE, FR, GB, IT, LI, LU, NL, SE.

1. 4-acylamino-1-azaadamantanes represented by general formula (I):

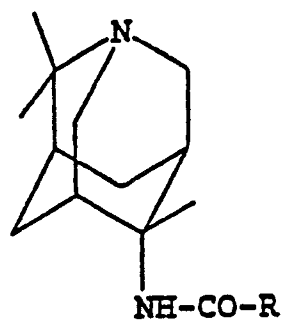

(I)

wherein R represents a phenyl group, a naphthyl group, a tolyl group, a phenethyl group, a benzyl group, a phenylpropyl group, a 2,2-diphenylethyl group, a 3,3-diphenyl-propyl group, said groups being possibly substituted by one or more substituents selected from an halogen atom or a lower alkyl group with 1 to 4 carbon atoms, or a hydroxy, methylenedioxy, methoxy, ethoxy, isopropoxy, amino, isopropylamino, dimethylamino, diethylamino, nitro, cyano, acetylamino, formyl, acetyl, trifluoromethyl and trichloromethyl groups, and acid salts thereof.

2. 4-acylamino-1-azaadamantanes according to claim 1, characterized in that R represents a phenyl, benzyl, phenethyl, phenylpropyl, 2,2-diphenylethyl, 3,3-diphenylpropyl, p-nitrophenyl, p-aminophenyl, p-acetylaminophenyl, p-methoxyphenyl, 3,4-dimethoxyphenyl, 3,4-dimethoxybenzyl, p-methoxybenzyl, p-methoxyphenethyl or 3,4-methylenedioxy-phenyl group.

3. 4-acylamino-1-azaadamantanes according to any of the preceding claims, characterized in that they are in the form of acid salt thereof.

4. Process for the preparation of the 4-acylamino-1-azaadamantane of claim 1, characterized in that it comprises reacting a 4-amino-1-aza-4,8,8-trimethyl-adamantane of general formula (II):

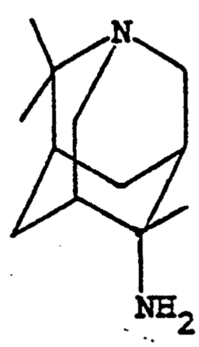

(II)

with an acylation agent in an appropriate solvent.

5. Process according to claim 4, characterized in that the acylation reaction is carried out by using an acid, an

acid chloride, an acid ester or an acid anhydride.

6. Process according to claim 4, characterized in that the acylation reaction is carried out by using an acid in the presence of N-hydroxysuccinimide and dicyclohexylcarbodiimide.

7. Process according to any of claims 4 to 6, characterized in that the solvent is an ether, tetrahydrofuran, dioxane, carbon tetrachloride, chloroform, methylene chloride or an ester.

8. Pharmaceutical compositions characterized in that they comprise a 4-acylamino-1-azaadamantane according to any of claims 1 to 4, or a pharmaceutically acceptable acid salt thereof, as an active ingredient, and possibly, a pharmaceutically acceptable carrier.

**Claims** for the contracting state AT.

1. Process for the preparation of 4-acylamino-1-azaadamantanes represented by general formula (I):

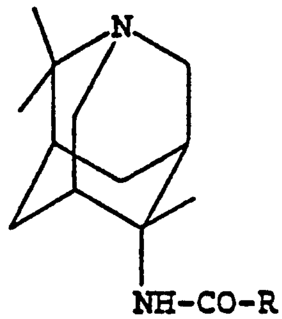

(I)

wherein R represents a phenyl group, a naphthyl group, a tolyl group, a phenethyl group, a benzyl group, a phenylpropyl group, a 2,2-diphenylethyl group, a 3,3-diphenylpropyl group, said groups being possibly substituted by one or more substituents selected from an halogen atom or a lower alkyl group with 1 to 4 carbon atoms, or a hydroxy, methylenedioxy, methoxy, ethoxy, isopropoxy, amino, isopropylamino, dimethylamino, diethylamino, nitro, cyano, acetylamino, formyl, acetyl, trifluoromethyl and trichloromethyl groups, characterized in that it comprises reacting a 4-amino-1-aza-4,8,8-trimethyl-adamantane of general formula (II)

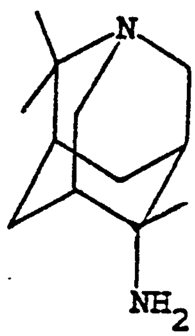

(II)

with an acylation agent in an appropriate solvent.

2. Process according to claim 8, characterized in that the acylation reaction is carried out by using an acid, an acid chloride, an acid ester or an acid anhydride.

3. Process according to claim 1, characterized in that the acylation reaction is carried out by using an acid in the presence of N-hydroxysuccinimide and dicyclohexylcarbodiimide.

4. Process according to any of claims 1 to 3, characterized in that the solvent is an ether, tetrahydrofuran, dioxane, carbon tetrachloride, chloroform, methylene chloride or an ester.